(19) Europäisches Patentamt
European Patent Office
Office européen des brevets

(11) Numéro de publication: **0 254 652 B1**

# (12) FASCICULE DE BREVET EUROPEEN

(45) Date de publication du fascicule du brevet: **10.10.90**

(51) Int. Cl.⁵: **C07C 45/45**, C07C 45/00, C07C 49/167, C07C 67/343, C07C 69/738

(21) Numéro de dépôt: **87420192.4**

(22) Date de dépôt: **09.07.87**

## (54) Procédé de préparation de perfluoro-alkylcétones.

(30) Priorité: **25.07.86 FR 8610988**

(43) Date de publication de la demande: **27.01.88 Bulletin 88/4**

(45) Mention de la délivrance du brevet: **10.10.90 Bulletin 90/41**

(84) Etats contractants désignés: **AT BE CH DE ES FR GB GR IT LI LU NL SE**

(56) Documents cités:
**EP-A- 0 028 778**
**US-A- 4 484 993**

**CHEMICAL ABSTRACTS, vol. 86, 1977, page 519, résumé no. 139560b, Columbus, Ohio, US; A. SEKIYA et al.: "Reaction of heptafluoro-1-methylethylzinc iodide with halides and anhydrides of carboxylic acids", & CHEM. LETT. 1977, (1), 81-4**
**RESEARCH DISCLOSURE, décembre 1973, pages 99-101, no. 11.662; "Synthesis of ethyl 4-haloacetoacetates"**

(73) Titulaire: **RHONE-POULENC CHIMIE, 25, quai Paul Doumer, F-92408 Courbevoie Cédex(FR)**

(72) Inventeur: **Francese, Catherine, Résidence des Coquelicots 111, rue de Chevilly, F-94240 L'Hay-les-Roses(FR)**
Inventeur: **Tordeux, Marc, 1, rue Seignelay, F-92330 Sceaux(FR)**
Inventeur: **Wakselman, Claude, 5, rue Chanese, F-75016 Paris(FR)**

(74) Mandataire: **Le Pennec, Magali et al, Rhone-Poulenc Chimie Service Brevets Chimie 25 Quai Paul Doumer, F-92408 Courbevoie(FR)**

EP 0 254 652 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation de perfluoroalkylcétones. Elle concerne plus particuliérement un procédé de préparation de trifluoropyruvate d'alkyle.

Les méthodes connues de préparation de perfluoroalkylcétones en particulier celle de préparation de l'hexafluoroacétone, sont décrites dans l'article de Krespan et Middleton paru dans "Fluorine Chemistry Reviews 1, 145-196 (1967)". On procède par exemple dans une première étape à une chloration de l'acétone puis dans une deuxième étape à un échange chlore-fluor par l'acide fluorhydrique à 300°C sur l'hexachloroacétone obtenue à la première étape.

La présente invention a permis de trouver un procédé tout-à-fait différent d'obtention des perfluoroalkylcétones caractérisé par le fait que l'on met en présence :

(1) un halogènure de perfluoroalkyle de formule générale Rf X (I) dans laquelle
- X représente Br ou I
- Rf représente un radical perfluoroalkyle dont la chaîne contient l à 12 atomes de carbone.
(2) du zinc
(3) un ester de formule générale (II)

$$R - \underset{\overset{\|}{O}}{C} - O - R' \qquad (II)$$

dans laquelle R représente un groupe cyano perfluoroalkyle, alkoxycarbonyle, R′ représente un groupe alkyle, aryle, alkylaryle, arylalkyle

dans un sovant aprotique dipolare et/ou une pyridine.

L'invention ne doit pas être limitée aux groupes définissant R préalablement cités mais à tous les groupes électroattracteurs ayant la même fonction que les groupes perfluoroalkyles, les groupes alcoxy carbonyles ou cyano.

Les iodures et bromures de perfluoroalkyle sont choisis de préférence parmi le bromure de trifluorométhyle et les iodures de perfluoroalkyle dont la chaîne alkyle perfluorée contient de 2 à 12 atomes de carbone. Cette préférence n'est pas due à une différence de réactivité des différents composés mais uniquement à une différence de coût. En effet, le bromotrifluorométhane est beaucoup moins cher que l'iodotrifluorométhane et inversement les iodures de perfluoroéthyle, perfluorobutyle... sont beaucoup moins chers que leurs analogues bromés.

On préfère utiliser le bromure de trifluorométhyle.

Parmi les esters de formule (II) on préfère utiliser ceux pour lesquels R représente un groupe perfluoroalkyle ou alkoxycarbonyle et en particulier les esters de l'acide trifluoroacétique ou de l'acide oxalique. Le radical R′ représente de préférence un groupe alkyle linéaire ou ramifié contenant 1 à 6 atomes de carbone, un groupe phényle

Parmi les composés de formule (II) on peut citer non limitativement : le trifluoroacétate d'éthyle et l'oxalate d'éthyle

Le zinc est avantageusement utilisé sous forme dispersée de façon à assurer le meilleur contact avec les gaz ou les liquides mis en oeuvre dans le procédé de l'invention. La forme et la dimension des particules de métal seront adaptées par l'homme de l'art à la réactivité des produits mis en oeuvre.

Pour une mise en oeuvre avantageuse de l'invention, on utilise un solvant aprotique polaire et/ou une pyridine.

Les solvants aprotiques polaires utilisables dans le procédé de l'invention sont choisis non limitativement parmi le diméthylformamide, le diméthylsulfoxyde, le diméthylacétamide, la N-méthylpyrolidone, l'hexaméthylphosphoramide.

On préfère utiliser le diméthylformamide et/ou une pyridine.

Parmi les pyridines on peut prendre l'une quelconque d'entre elles substituées ou non par un ou plusieurs groupes alkyles telles que la méthyl ou diméthylpyridine, mais on préfère utiliser la pyridine non substituée.

La pyridine permet de réduire considérablement le temps d'induction de la réaction.

Selon une mise en oeuvre avantageuse de l'invention, on utilise de préférence un rapport molaire du zinc à l'ester supérieur ou égal à 1 et inférieur à 2 et un rapport molaire de l'iodure ou du bromure de perfluoroalkyle à l'ester supérieur ou égal à 1. Si l'halogénure de perfluoroalkyle est mis en excès et que l'halogénure utilisé est le bromure de trifluorométhyle, celui-ci sera aisément recyclé car il se présente sous forme gazeuse.

Pour une meilleure mise en oeuvre de la réaction, on préfère maintenir une température comprise entre -20°C et 100°C.

On opère de préférence en l'absence d'oxygène.

La pression est de préférence supérieure ou égale à la pression atmosphérique et notamment comprise ente 1 et 10 bars. Parmi les produits obtenus selon le procédé de l'invention, on peut citer : le trifluoropyruvate d'éthyle et l'hexafluoroacétone.

Les produits issus de la présente invention sont utilisés dans la synthèse de copolymères modifiés "Fluorine Chemistry Reviews 1, 145-196, (1967)" ou comme intermédiaires de synthèse dans la fabrication d'hétérocycles [Journal of Fluorine Chemistry, 30 (1986) 463-468].

L'invention sera plus complètement décrite à l'aide des exemples suivants qui ne doivent pas être considérés comme limitatifs du domaine de l'invention.

EXEMPLES

Exemple 1 : trifluoroacétate d'éthyle - $CF_3Br$

Dans un flacon en verre épais, on met 15 ml de pyridine, 5 g de trifluoroacétate d'éthyle (0,0352 mole) et 2,5 g de zinc en poudre (0,0385 mole).

Le flacon est placé dans un appareil de Parr. On y fait le vide puis on introduit du bromotrifluorométhane jusqu'à une pression de 3 bars ; celle-ci étant maintenue entre 4 et 2,6 bars pendant la réaction. On agite pendant tout le temps de la réaction.

On filtre puis on hydrolyse par 50 ml d'acide chlorhydrique à 10 % an agitant 30 mn.

Après extraction à l'éther, lavage à l'eau, séchage sur sulfate de magnésium et évaporation du solvant, l'hexafluoroacétone est distillée sous forme d'hydrate - Eb = 34 - 36°C/20 mmHg.

On obtient 3,5 g (54 %).

($Eb_{litt.}$(5) =55 - 56°C/80 mmHg)

RMN$^{19}$F ($CFCl_3$ EXT.) : -83,3 ppm (s, $CF_3$).

Exemple 2 : Oxalate d'éthyle - $CF_3Br$

Dans un flacon en verre épais, on met 50 ml de pyridine, 20 ml d'oxalate d'éthyle (0,147 mole) et 10 g de zinc en poudre (0,154 mole).

Le flacon est placé dans un appareil de Parr. On y fait le vide puis on introduit du bromotrifluorométhane jusqu'à une pression de 3,6 bars ; on agite pendant tout le temps de la réaction en maintenant la pression entre 4 et 2,6 bars.

On filtre puis on hydrolyse par 50 ml d'acide chlorhydrique à 10 % glacé en agitant 30 mn.

Après extraction à l'éther, lavage à l'eau, séchage sur sulfate de magnésium et évaporation du solvant, le trifluoropyruvate d'éthyle est obtenu sous forme d'hydrate.

La déshydratation est ensuite effectuée avec de l'acide sulfurique concentré, à température ambiante, et conduit à 9,5 g de trifluoropyruvate d'éthyle (38 %) piégés sous vide à -78°C ($Eb_{litt.}$= 89°C).

RMN$^{19}$F (CF $Cl_3$ EXT.) = -75,7ppm (s,$CF_3$)

RMN'H (TMS int.) = 4,5 ppm (q, $CH_2$) ; 1,4 ppm (t, $CH_3$)

Exemple 3 : Oxalate d'éthyle - $C_6F_{13}I$

Dans un ballon on met 10 ml de pyridine, 3 ml d'oxalate d'éthyle (0,0221 mole) et 1,5 g de zinc en poudre (0,023 mole).

On purge à l'Argon et on additionne ensuite 10 g de iodo-1 perfluoro n-hexane (0,022 mole) en agitant. La réaction est exothermique.

On filtre puis on hydrolyse par 20 ml d'acide chlorhydrique à 10 % glacé en agitant 30 mn.

Après extraction à l'éther, lavage à l'eau, séchage sur sulfate de magnésium et évaporation du solvant, l'oxo-2 tridécafluorooctanoate d'éthyle est distillé - Eb = 52-58°C/20 mmHg. On obtient 1,8 g (20 %).

RMN$^{19}$F ($CFCl_3$ EXT.) : - 80,7 ppm (t.t., $CF_3$) ;
- 117 à - 123 ppm (m, 8 F) ;
- 125 à - 127 ppm (m, 2 F).

**Revendications**

1. Procédé de préparation de perfluoroalkylcétones caractérisé en ce que l'on met en présence :
(1) un halogènure de perfluoroalkyle de formule générale Rf X (I) dans laquelle
- X représente Br ou I
- Rf représente un radical perfluoroalkyle dont la chaîne contient 1 à 12 atomes de carbone.
(2) du zinc
(3) un ester de formule générale (II)

$$R-\underset{\underset{O}{\|}}{C}-O-R' \qquad (II)$$

dans laquelle R représente un groupe cyano, perfluoroalkyl, alkoxycarbonyle, R′ représente un groupe alkyle, aryle, alkylaryle, arylalkyle
dans un solvant aprotique dipolaire et/ou une pyridine.

2. Procédé selon la revendication 1 caractérisé en ce que le composé de formule (I) est le bromure de trifluorométhyle.

3. Procédé selon la revendication 1 caracatérisé en ce que l'ester de formule (II) est un ester d'un acide perfluoroalkylé.

4. Procédé selon la revendication 1 caractérisé en ce que l'ester de formule (II) est un ester de l'acide trifluoroacétique ou de l'acide oxalique.

5. Procédé selon la revendication 1 caractérisé en ce que le solvant aprotique dipolaire est le diméthylformamide.

6. Procédé selon la revendication 1 caractérisé en ce que le solvant choisi est la pyridine.

7. Procédé selon la revendication 1 caractérisé en ce que la température de réaction est comprise entre -20°C et 100°C.

8. Procédé selon la revendication 1 caractérisé en ce que la pression réactionnelle est supérieure ou égale à la pression atmosphérique et de préférence inférieure à 10 bars.

**Patentansprüche**

1. Verfahren zur Herstellung von Perfluoralkylketonen, dadurch gekennzeichnet, daß man in einem aprotischen dipolaren Lösungsmittel und/oder in einem Pyridin miteinander reagieren läßt:
(1) Ein Perfluoralkylhalogenid der allgemeinen Formel Rf X (I), in der
– X Brom oder Jod darstellt
– Rf einen Perfluoralkylrest darstellt, dessen Kette zwischen 1 und 12 Kohlenstoffatome enthält.
(2) Zink
(3) Einen Ester der allgemeinen Formel (II)

$$R-\underset{\underset{O}{\|}}{C}-O-R' \qquad (II)$$

in der R eine Cyan-, Perfluoralkyl- oder Alkoxycarbonylgruppe und R′ eine Alkyl-, Aryl-, Alkylaryl- oder Arylakylgruppe darstellt.

2. Verfahren nach Anspruch 1, dadurch gekennzeichnet, daß die Verbindung der Formel (I) das Trifluorbrommethan ist.

3. Verfahren nach Anspruch 1, dadurch gekennzeichnet, daß der Ester der Formel (II) ein Ester einer perfluoralkylierten Säure ist.

4. Verfahren nach Anspruch 1, dadurch gekennzeichnet, daß der Ester der Formel (II) ein Ester der Trifluoressigsäure oder der Oxalsäure ist.

5. Verfahren nach Anspruch 1, dadurch gekennzeichnet, daß das aprotische dipolare Lösungsmittel Dimethylformamid ist.

6. Verfahren nach Anspruch 1, dadurch gekennzeichnet, daß das gewählte Lösungsmittel Pyridin ist.

7. Verfahren nach Anspruch 1, dadurch gekennzeichnet, daß die Reaktionstemperatur zwischen –20°C und 100°C liegt.

8. Verfahren nach Anspruch 1, dadurch gekennzeichnet, daß der Reaktionsdruck größer oder gleich dem atmosphärischen Druck und vorzugsweise niedriger als 10 bar ist.

**Claims**

1. Process for the preparation of perfluoroalkyl ketones, characterized in that the following are brought into contact:
(1) a perfluoroalkyl halide of general formula Rf X (I) in which
– X represents Br or I, and
– Rf represents a perfluoroalkyl radical which contains 1 to 12 carbon atoms in its chain
(2) zinc, and
(3) an ester of general formula (II)

$$R - \underset{\underset{O}{\|}}{C} - O - R' \qquad (II)$$

in which R represents a cyano, perfluoroalkyl or alkoxycarbonyl group and R', represents an alkyl, aryl, alkylaryl or arylalkyl group in a dipolar aprotic solvent and/or a pyridine.

2. Process according to Claim 1, characterized in that the compound of formula (I) is trifluoromethyl bromide.

3. Process according to Claim 1, characterized in that the ester of formula (II) is an ester of a perfluoroalkylated acid.

4. Process according to Claim 1, characterized in that the ester of formula (II) is a trifluoroacetic acid or oxalic acid ester.

5. Process according to Claim 1, characterized in that the dipolar aprotic solvent is dimethylformamide.

6. Process according to Claim 1, characterized in that the solvent chosen is pyridine.

7. Process according to Claim 1, characterized in that the reaction temperature is between –20°C and 100°C.

8. Process according to Claim 1, characterized in that the reaction pressure is higher than or equal to atmospheric pressure and preferably lower than 10 bars.